# EUROPEAN PATENT SPECIFICATION

(11) **EP 2 731 660 B1**
(45) Date of publication and mention of the grant of the patent: **31.05.2017**
(21) Application number: 12737944.4
(22) Date of filing: 27.06.2012
(51) Int. Cl.: A61M 25/10

(54) **DRUG ELUTION MEDICAL DEVICE**
MEDIKAMENTENBESCHICHTETES MEDIZINPRODUKT
DISPOSITIF MÉDICAL À ÉLUTION MÉDICAMENTEUSE

(30) Priority: 12.07.2011 US 201161506818 P
(43) Date of publication of application: 21.05.2014
(73) Proprietor: Boston Scientific Scimed, Inc., Maple Grove, MN 55311-1566 (US)
(72) Inventor: FOLAN, Martyn, Loughrea Co. Galway (IE); HORGAN, Fergal, Shrule Co. Mayo (IE); TURKINGTON, Marie, Shrule Co. Mayo (IE)
(74) Representative: Peterreins Schley
(86) International application number: PCT/US2012/044428
(87) International publication number: WO 2013/009476

(56) References cited:
- US-A1- 2011 137 155

## Description

### TECHNICAL FIELD

This disclosure relates to medical devices for therapeutic agent delivery, and more particularly, to medical devices containing biodegradable polymer layers for therapeutic agent delivery.

### BACKGROUND

The body includes various passageways such as blood vessels (e.g., arteries) and lumens. These passageways sometimes become occluded (e.g., by a tumor or plaque). To widen an occluded vessel or lumen, balloon catheters can be used, e.g., in angioplasty.

A balloon catheter can include an inflatable and deflatable balloon carried by a long and narrow catheter body. The balloon can be initially folded around the catheter body to reduce the radial profile of the balloon catheter for easy insertion into the body.

During use, the folded balloon can be delivered to a target location in the vessel, e.g., a portion occluded by plaque, by threading the balloon catheter over a guide wire placed in the vessel. The balloon is then inflated, e.g., by introducing a fluid into the interior of the balloon. Inflating the balloon can radially expand the vessel so that the vessel can permit an increased rate of blood flow. After use, the balloon is typically deflated and withdrawn from the body.

In some instances, percutaneous coronary and peripheral interventions that depend upon mechanical dilatation of the artery or ablation of atherosclerotic plaque can be associated with plaque fracture, intimal splitting and/or localized medial dissection. The plaque fracture, intimal splitting and/or localized medial dissection can extend into a lumen's lining for varying distances, and may even extend through the adventitia resulting in perforation of a lumen (e.g., a blood vessel). US2009187144 and US2011137155 disclose two exemplary balloon catheter provided with drug delivery capabilities.

### SUMMARY

The invention is defined as in claims 1,15. Therapeutic agents can be delivered to the vessels and lumens of the body (body lumens) via medical devices, such as endoprostheses. The present disclosure is based, at least in part, on an endoprosthesis such as a sleeve that can, for example, provide effective temporary relief of a vascular dissection (e.g., as a dissection closure device) that has been generated during a clinical procedure by sealing off the generated breach, and that can allow a vessel lumen to remain available (e.g., by maintaining an open vessel lumen) for alternative treatment of the dissection. For example, the sleeve can provide continued clinical interventions in vascular areas that are adjacent to a dissection site, even after removal of a carrier device (e.g., an expandable balloon). The sleeve can deliver a predetermined dosage of a coagulant and/or other therapeutic agent. In some embodiments, a stent may be co-administered along with the sleeve (e.g., the sleeve may be disposed on an abluminal surface of the stent).

Accordingly, in one aspect, the disclosure features a medical device, including a tubular layer having an adluminal surface and an abluminal surface, the layer including a first polymer and a biologically active agent; a tissue-adhesive region disposed on the outer abluminal surface; and a mask region including a second polymer disposed on the inner adluminal surface.

In another aspect, the disclosure features a method of making a medical device, including (a) applying a layer including a second polymer to a non-stick substrate to form a substantially non-porous mask region; (b) applying a tubular layer including a first polymer and biologically active agent to the mask region; (c) applying a tissue-adhesive region to the tubular layer to form a sleeve; (d) removing the sleeve from non-stick substrate; and (e) disposing the sleeve over an expandable balloon coated with a first release agent.

Embodiments of the above-mentioned medical devices can have one or more of the following features.

The device can be biodegradable (e.g., biodegradable within a within a period of about one month to about twelve months). The first and second polymer can each be selected from any of the polymers described, *infra.* For example, the first and second polymers can each be independently selected from the group consisting of polyurethanes, polyethylene, polylactic acid, polyglycolic acid, polylactic-co-glycolic acid, poly-DL-lactide, and any combination thereof.

In some embodiments, the mask region is non-porous.

In some embodiments, the tissue-adhesive region is configured on the abluminal surface as a plurality of strips, a plurality of dots, a continuous layer, a matrix mesh, a plurality of longitudinal strips, a plurality of circumferential strips, or any combination thereof. The tissue-adhesive region can include a repeating pattern of dots, strips, or both. The tissue- adhesive region can be disposed at a first end and second end of the tubular layer. The tissue adhesive region can be disposed over about 5 percent or more of the abluminal surface of the tubular layer. The tissue-adhesive region can include any of the tissue-adhesive substances described, *infra.* For example, the tissue-adhesive region can include polyethylene glycol, dextran aldehyde, amino acid-based adhesives, adhesive surface proteins, microbial surface components-recognizing adhesive matrix molecules ("MSCRAMMS"), fatty acid modified PLA, fatty acid modified PLGA, gel particles, poly(N-isopropylacrylamide) gel particles, or any combination thereof.

The medical device can be disposed on an expandable balloon. The medical device can further include a release region disposed between the medical device and the surface of the expandable balloon. The release region can be adherent to the expandable balloon. In some embodiments, the release region can be disposed over about 5 percent or more of the adluminal surface area of the mask layer. The release layer can include contrast agents (e.g., iopromide), proteins (e.g., gelatin-based glues, protein-based adhesives), synthetic glues (e.g., cyanoacrylates), or any combination thereof.

The biologically active agent can include any of the biologically active agents decribed, *infra.* For example, the biologically active agent can include antianginals, analgesics, nitrates, beta blockers, calcium channel blockers, paracetamol, NSAIDs, COX-2 inhibitors, flupirtine, coagulant promotors, procoagulants, desmopressin, paclitaxel, everolimus, sirolimus, zotarolimus, biolimus A9, and/or any combination thereof.

In some embodiments, the medical device can include a vascular sleeve.

Embodiments of the above-mentioned medical devices can have one or more of the following advantages.

In some embodiments, the medical device provides improved targeted treatment of acute iatrogenic vascular dissections or perforations. In some embodiments, the medical device delivers a therapeutic agent dosage to the site of acute vascular dissections or perforations in a more efficacious manner, as the medical device can maintain a therapeutic agent in close proximity to a vascular lining, thereby improving cellular response and vessel repair. The medical device can provide increased efficiency for drug delivery, decreased costs, and ease of intervention over conventional treatment methods for vascular dissections or perforations. In some embodiments, the medical device can minimize the overall clinical procedural time while reducing the requirement for additional interventional procedures, such as the multiple implantations of stents and/or multiple administrations of drug-eluting balloons.

In some embodiments, the medical device can be used in and/or allows the performance of more challenging interventions, such as in interventions for more tortuous anatomy, for treating distal lesions, or for treating bifurcations, since the risk of creation of unmanageable dissections can be greatly reduced. For example, the medical device can manage and treat dissections by allowing the primary intervention to be completed in a single session, while minimizing the need (e.g., eliminating the need) for the use of tertiary equipment and interventions, thereby also providing cost benefits. Examples of tertiary equipment and interventions include stenting or scenarios where multiple devices may be required for treatment of a vascular dissection and/or perforation. In some embodiments, the medical device is capable of delivering more than one therapeutic agent.

The medical device can be scaled in size for peripheral or coronary interventions. For example, the medical device can be larger for peripheral vessels. The medical device can be used with existing balloon technologies. The medical device can be relatively easily made with conventional spray technologies.

The medical devices of the present disclosure include implantable and insertable medical devices that are used for the treatment of various mammalian tissues and organs. As used herein, "treatment" refers to the prevention of a disease or condition, the reduction or elimination of symptoms associated with a disease or condition, or the substantial or complete elimination of a disease or condition. Subjects are vertebrate subjects, more typically mammalian subjects including human subjects, pets and livestock.

As used herein, a "layer" of a given material is a region of that material whose thickness is substantially less than its length and width. Layers can be in the form of open structures (e.g., sheets, in which case the thickness of the layer is substantially less than the length and width of the layer), and partially closed structures (e.g., open tubes, in which case the thickness of the layer is substantially less than the length and diameter of tube).

As used herein, a polymer is "biodegradable" if it undergoes bond cleavage along the polymer backbone *in vivo,* regardless of the mechanism of bond cleavage (e.g., enzymatic breakdown, hydrolysis, oxidation, etc.). A biodegradable polymer includes "bioerosion" or "bioabsorption" of a polymer-containing component of a medical device (e.g., a polymer-containing layer), as well as other *in vivo* disintegration processes such as dissolution, etc. Biodegradability is characterized by a substantial loss *in vivo* over time (e.g., the period that the device is designed to reside in a patient) of the original polymer mass of the component. For example, losses may range from 50% to 75% (e.g., to 90%, to 95%, to 97%, to 99%, or more) of the original polymer mass of the device component. Bioabsorption times may vary widely, for example, bioabsorption times can range from several hours to approximately one year.

The details of one or more embodiments of the disclosure are set forth in the accompanying drawings and the description below. Other features, objects, and advantages of the disclosure will be apparent from the description and drawings, and from the claims.

### DESCRIPTION OF DRAWINGS

FIG. 1A is a side view of an embodiment of a medical device;
FIG. 1B is a cross-sectional view of an embodiment of a medical device;
FIG. 1C is an enlarged cross-sectional view of an embodiment of a medical device;
FIGS. 2A-2E are side views of an embodiment of a medical device during deployment;
FIGS. 3A-3C are enlarged cross-sectional views of an embodiment of a medical device during deployment;
FIGS. 4A-4C show an embodiment of a method of manufacture of a medical device; and
FIGS. 5A-5C show an embodiment of a method of manufacture of a medical device.

Like reference symbols in the various drawings indicate like elements.

### DETAILED DESCRIPTION

In embodiments, this disclosure relates to a medical device, such as a sleeve that can elute a therapeutic agent to, for example, peripheral and/or cardiovascular body lumen walls. The sleeve can be carried by an inflatable carrier balloon. When the balloon is inflated in a vascular lumen, the sleeve can intimately contact the vasculature and adhere to the interior of a lumen's treatment site (e.g., endothelial cells lining a vasculature area surrounding a dissection or perforation). Upon subsequent balloon deflation and withdrawal, the sleeve can remain at the treatment site.

In some embodiments, the sleeve can provide temporary relief of a vascular dissection that has been generated, for example, during a clinical procedure, by sealing off a generated breach. The sleeve can allow the vessel lumen to remain available for further alternative treatment of the dissection (e.g., placement of a stent) and/or continued clinical interventions in vascular areas that are adjacent to the dissection site. To reduce blood loss and the likelihood of further deterioration of a body lumen, the sleeve can deliver a coagulant or other therapeutic agent to the site of a vascular dissection or perforation.

The sleeve can effectively seal an area of vascular dissection or perforation without the need for prolonged balloon inflation. The sleeve can effectively seal an area of vascular dissection or perforation, and may not require that a patient be removed from a systemic course of anticoagulation treatment that, for example, may have been previously administered. The sleeve can offer increased predictability to therapeutic agent delivery, it can help to alleviate the acute and chronic conditions associated with coronary artery disease (CAD) and peripheral arterial disease (PAD), and/or it can minimize the number of procedures at a given site of a weakened body lumen.

In some embodiments, the sleeve can be used to treat sites where stent implantation is not desirable, such as small vessels. The sleeve can be used instead of or in addition to drug-eluting stents. In some embodiments, the sleeve can homogenously deliver therapeutic agents to a vascular site, which can decrease the likelihood of vascular stiffening, while maintaining the accessibility of the blood vessel for re-intervention and decreasing the likelihood of restenosis, when compared to conventional interventions, such as stent implantation and balloon angioplasty.

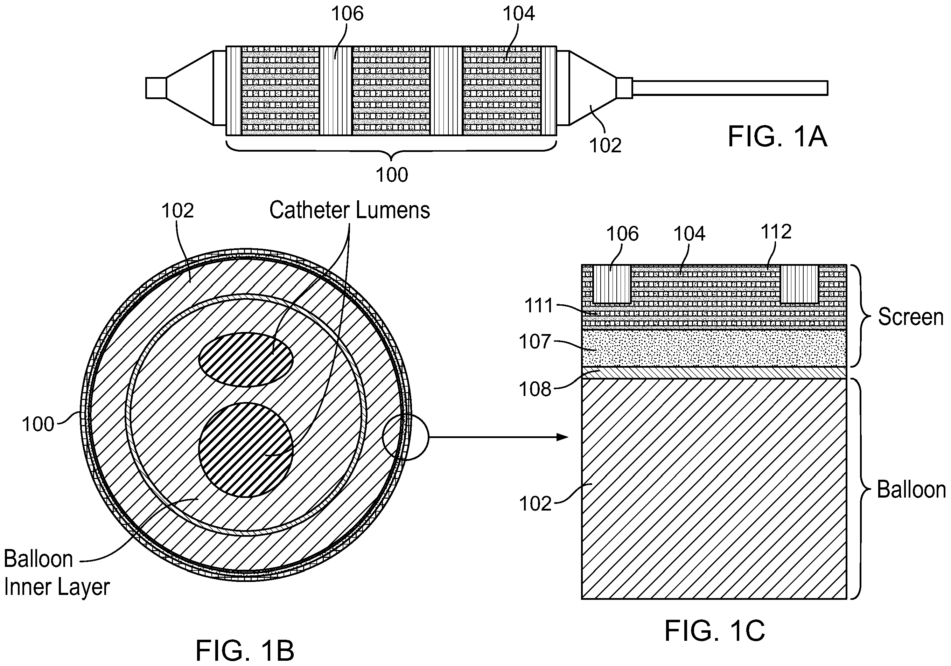

Referring to FIG. 1A, the sleeve can have a tubular construction, in the form of a sleeve 100. Sleeve 100 can be used in conjunction with a carrier balloon expandable catheter 102 and can be left within a blood vessel following balloon deployment and withdrawal. The sleeve can deliver therapeutic agents during or in addition to angioplasty procedures, and can provide prolonged drug delivery to a body lumen wall after angioplasty procedures. In some embodiments, the sleeve can provide one or more therapeutic agents, which can elute over specific time frames and/or in particular sequences. The sleeve can decrease a therapeutic agent dosage, decrease the likelihood of drug loss from a body lumen wall, or the premature drug loss from a drug delivery device, for example, when a drug delivery device is maneuvered through a blood vessel. The sleeve can provide drug delivery in a temporary capacity. For example, a sleeve can be degradable (e.g., after therapeutic agent delivery and dissection stabilization has been achieved), which can decrease the likelihood of device-related thrombosis or embolism, while providing therapeutic agent treatment for vascular inflammation and delayed re-endothelialization.

Referring to FIGS. 1B and 1C, a sleeve 100 can include a biodegradable substrate layer 104 and one or more tissue adhesive region(s) 106 on an abluminal surface of the sleeve. One or more mask region(s) 107 can be disposed on an adluminal surface of the sleeve. The mask region can be biodegradable. One or more balloon release region(s) 108 can be disposed on an adluminal surface of the sleeve, between mask region 107 and balloon 102. Biodegradable substrate layer 104 can provide structural shape to the sleeve, and referring to FIG. 1C, can include a polymer matrix 111 which can contain a therapeutic agent 112. Biodegradable substrate layer 104 can protect the therapeutic agent, for example, until a target treatment site is reached. The polymer matrix 111 can elute the therapeutic agent in a controlled manner. Elution of the therapeutic agent can occur preferentially to the surface of the blood vessel to which the sleeve is in contact. For example, mask region 107 can be relatively impermeable and can have the same surface area as an overlying biodegradable substrate layer, such that the therapeutic agent can elute to the blood vessel surface contacting the sleeve, but not to the body lumen. After drug elution is completed during a predetermined time frame, biodegradable substrate layer 104 can degrade in a controlled manner leaving little or no residual components at the treatment site. In some embodiments, after drug elution is completed during a predetermined time frame, mask region 107 can degrade in a controlled manner leaving little or no residual components at the treatment site. In some embodiments, the tissue adhesive regions 106 can also degrade, be absorbed, or be excreted in a controlled manner, leaving little or no residual components at the treatment site.

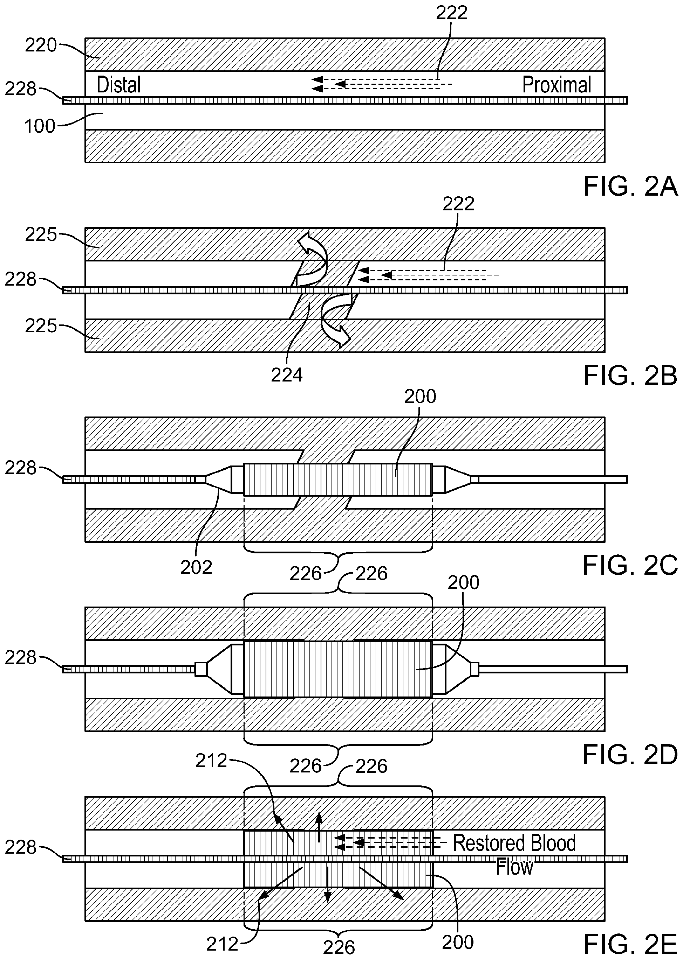

In some embodiments, referring to FIGS. 2A-2E, dissections can be caused by the interaction of minimally invasive equipment (guidewires, stents, filters, POBA, cutting balloons, ablation devices etc.) at the treatment site of the vasculature. Prior to dissection, the vasculature 220 can appear normal with identifiable blood flow 222 (FIG. 2A). Once a dissection 224 has been caused, depending on the severity of the breach, the blood loss 225 into the surrounding tissue 226 can be significant leading to trauma conditions, such as collapse of the distal vasculature, irreversible tissue death, or myocardial infarction. As the dissection 224 can usually be caused in the course of a clinical procedure, a guidewire 228 can still be in place across the dissection, spanning the vascular portions surrounding the dissection (FIG. 2B). A low profile sleeve 200 can then be traversed across this existing guidewire or over a newly introduced guidewire in such a manner to be positioned across the dissection location (FIG. 2C). The sleeve 200 is then deployed via an expandable balloon 202. Upon deployment, the sleeve 200 comes into intimate contact with the vasculature 226 surrounding the dissection. The sleeve can adhere to the endothelial cell (EC) tissue lining the vasculature of the targeted site 226 (FIG. 2D).

Upon subsequent balloon deflation and carrier device withdrawal, the sleeve 202 can remain at the treatment site, leaving a functional lumen for continued interventional procedures (e.g., at a distal vascular location to the treatment site). As the therapeutic agent is in intimate contact with the treatment site, a therapeutic agent 212 can be more effectively delivered to the treatment site 226 (FIG. 2E). The sleeve can be formed in a manner that the therapeutic agent (e.g., coagulant) can be exclusively exposed to the breach location on the outer surface of the device and can be effectively masked from the vascular lumen where the therapeutic agent would be less advantageous.

Post-procedure, the treatment site 226 can continue to benefit from the availability of the therapeutic agent 212 through the remaining sleeve over a predetermined time course. As this time course ends, the sleeve 200 can degrade, returning the native vasculature to a state which has improved vascular clearance to blood flow.

In some embodiments, when implanted in a body lumen, the sleeve is biodegradable within a period of about one month (e.g., about two months, about six months, or about ten months) to about twelve months (e.g., about ten months, about six weeks, about one month). In some embodiments, the sleeve can degrade within a period of several minutes (e.g., about two minutes, about five minutes, about ten minutes, about 20 minutes, about an hour, about five hours, about 12 hours, about 24 hours, or more). The biodegradability of the sleeve can depend on the polymers of the biodegradable substrate layer, the tissue adhesive region, and the therapeutic agent.

In some embodiments, in a sleeve including a biodegradable polymer, such as PLGA, the copolymer ratio of the monomers (e.g., lactide to glycolide) can determine the rate of polymer degradation. For example, higher lactide content can lead to slower degradation. In some embodiments, molecular weight can affect the degradation. For example, a lower molecular weight can lead to faster degradation (e.g., when the molecular weight is below the range where T_{g} is affected). In some embodiments, a polymer end group can be used to control the rate of degradation. For example, an alkyl end group associated with a co-polymer, such as PDLLA, can result in slower degradation than a polymer with an acid end group, such as in PLGA. In some embodiments, crystallinity, drug percent loading, and/or other additives can also affect degradation.

In some embodiments, when a sleeve has a thickness of less than or equal to about 200 µm (e.g., less than or equal to about 150 µm, less than or equal to about 100 µm), the sleeve thickness can have a minimal impact on degradation. In some embodiments, when a sleeve has a thickness of greater than about 200 µm (e.g., greater or equal to about 350 µm, greater than or equal to about 400 µm), oligomers can have a slower rate of diffusion out of a polymer layer and result in an accumulation of acidic degradation products at a center layer of sleeve, which can contribute to autocatalytic degradation (e.g. heterogeneous degradation). As an example, *in vitro* mass loss in bio-relevant media at 37 °C for a sleeve of about 200 µm thick including 85/15 lactide:glycolide PLGA co-polymer can demonstrate greater than 85 % mass loss in less than about 180 days.

Polymers which may be used to form biodegradable substrate layers include synthetic and natural biodegradable polymers. Synthetic biodegradable polymers include polyesters, for example, selected from homopolymers and copolymers of lactide, glycolide, and epsilon-caprolactone, including poly(L-lactide), poly(D, L-lactide), poly(lactide-co-glycolides) such as poly(L-lactide-co-glycolide) and poly(D, L-lactide-co-glycolide), polycarbonates including trimethylene carbonate (and its alkyl derivatives), polyphosphazines, polyanhydrides, polyorthoesters, and biodegradable polyurethanes. Natural biodegradable polymers include proteins, for example, selected from fibrin, fibrinogen, collagen and elastin, and polysaccharides, for example, selected from chitosan, gelatin, starch, and glycosaminoglycans such as chondroitin sulfate, dermatan sulfate, keratin sulfate, heparin, heparan sulfate, and hyaluronic acid. In some embodiments, the polymers can include one or more of alginate, dextran, chitin, cotton, polylactic acid-polyethylene oxide copolymers, cellulose, and chitins. Blends of the above natural and synthetic polymers may also be employed.

In some embodiments, polymers suitable for biodegradable substrate layers can include without limitation polyurethane and its copolymers, silicone and its copolymers, ethylene vinyl-acetate, polyethylene terephthalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polycarbonate, poly(glycolide-lactide) copolymer, Tecothane, PEBAX, polyethylene, polylactic acid, poly(γ-caprolactone), poly(γ-hydroxybutyrate), polydioxanone, poly(γ-ethyl glutamate), polyiminocarbonates, poly(ortho ester), and/or polyanhydrides. Additional polymeric materials are described, for example, in U.S. Patent No. 5,650,234 and 5,463,010, herein incorporated in their entirety. Blends of the above polymers may also be employed.

In some embodiments, biodegradable substrate layer 104 includes biodegradable materials, such as polyglycolic acid, polylactic acid, poly(lactic-co-glycolic acid), poly-DL-lactide, and/or other known degradable polymers. Biodegradable substrate layer 104 can also include non-biodegradable materials, such as Tecothane, PEBAX, and/or polyethylene. In some embodiments, biodegradable substrate layer 104 contains, for example, from 1 to 100 wt% (e.g., from 25 to 50 wt %, from 25 to 75 wt %, from 75 to 90 wt%, from 85 to 99 wt %, from 90-99 wt %, from to 95 to 99 wt%, 100 wt%) of one or more biodegradable polymers. In some embodiments, the weight percent of biodegradable material can be 80 % or more (e.g., 90 % or more, 95 % or more, or 99 % or more) of the total polymer contained by biodegradable substrate layer 104. The weight percent of non-biodegradable material can be 20 % or less (e.g., 10 % or less, 5 % or less, or 1 % or less) of the total polymer contained by biodegradable substrate layer 104. In some embodiments, incorporation of a non-biodegradable material can provide increased stability to the resulting material, such that the biodegradable substrate layer can have increased resistance to degradation (e.g., during storage, in humid environments). In some embodiments, biodegradable substrate layer 104 can be in the form of a fibrous scaffold with an open porous structure that encourages three-dimensional migration and proliferation of cells within the fibrous scaffold. Examples of biodegradable substrate layer 104 include non-porous layers and porous layers.

In some embodiments, biodegradable substrate layer 104 can have a thickness of about 5 nm or more (e.g., about 10 nm or more, about 20 nm or more, about 50 nm or more, about 100 nm or more, about 500 nm or more, about one micron or more, about 10 microns or more, about 25 microns or more, about 50 microns or more, or about 70 microns or more) and/or about 80 µm or less (e.g., about 70 microns or less, about 50 microns or less, about 25 microns or less, about 10 microns or less, about one micron or less, about 500 nm or less, about 100 nm or less, about 50 nm or less, about 20 nm or less, or about 10 nm or less). In some embodiments, biodegradable substrate layer 104 can be a uniform layer, or patches that may or may not be interconnected. The biodegradable substrate layer can define the length and expanded diameter of the sleeve. For example, the biodegradable substrate layer can have a length of about 6 mm or more (e.g., about 10 mm or more, about 20 mm or more, about 30 mm or more, or about 35 mm or more) and/or about 40 mm or less (e.g., about 35 mm or less, about 30 mm or less, about 20 mm or less, or about 10 mm or less). In some embodiments, the biodegradable substrate layer can have an expanded diameter of about 2 mm or more (e.g., about 3 mm or more, about 4 mm or more, or about 5 mm or more) and/or about 6 mm or less (e.g., about 5 mm or less, about 4 mm or less, or about 3 mm or less).

The mask regions can be relatively impermeable to adluminal therapeutic agents. Polymers which may be used to form the mask region include synthetic and natural biodegradable polymers. Synthetic biodegradable polymers include polyesters, for example, selected from homopolymers and copolymers of lactide, glycolide, and epsilon-caprolactone, including poly(L-lactide), poly(D, L-lactide), poly(lactide-co-glycolides) such as poly(L-lactide-co-glycolide) and poly(D, L-lactide-co-glycolide), polycarbonates including trimethylene carbonate (and its alkyl derivatives), polyphosphazines, polyanhydrides and polyorthoesters. Natural biodegradable polymers include proteins, for example, selected from fibrin, fibrinogen, collagen and elastin, and polysaccharides, for example, selected from chitosan, gelatin, starch, and glycosaminoglycans such as chondroitin sulfate, dermatan sulfate, keratin sulfate, heparin, heparan sulfate, and hyaluronic acid. In some embodiments, the polymers can include one or more of alginate, dextran, chitin, cotton, polylactic acid-polyethylene oxide copolymers, cellulose, and chitins. Blends of the above natural and synthetic polymers may also be employed.

In some embodiments, polymers suitable for the mask region include without limitation polyurethane and its copolymers, silicone and its copolymers, ethylene vinyl-acetate, polyethylene terephthalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polycarbonate, poly(glycolide-lactide) copolymer, Tecothane, PEBAX, polyethylene, polylactic acid, poly(γ-caprolactone), poly(γ-hydroxybutyrate), polydioxanone, poly(γ-ethyl glutamate), polyiminocarbonates, poly(ortho ester), and/or polyanhydrides. Blends of the above polymers may also be employed.

In some embodiments, mask region 107 includes biodegradable materials, such as polyglycolic acid, polylactic acid, poly(lactic-co-glycolic acid), poly-DL-lactide, and/or other known degradable polymers. Mask region 107 can also include non-biodegradable materials, such as Tecothane, PEBAX, and/or polyethylene. In some embodiments, mask region 107 contains, for example, from 1 to 100 wt% (e.g., from 25 to 50 wt %, from 25 to 75 wt %, from 75 to 90 wt%, from 85 to 99 wt %, from 90-99 wt %, from to 95 to 99 wt%, 100 wt%) of one or more biodegradable polymers. In some embodiments, the weight percent of biodegradable material can be 80 % or more (e.g., 90 % or more, 95 % or more, or 99 % or more) of the total polymer contained by mask region 107. The weight percent of non-biodegradable material can be 20 % or less (e.g., 10 % or less, 5 % or less, or 1 % or less) of the total polymer contained by mask region 107. In some embodiments, incorporation of a non-biodegradable material can provide increased stability to the resulting material, such that the mask region can have increased resistance to degradation (e.g., during storage, in humid environments). In some embodiments, mask region 107 can have an increased amount of non-biodegradable material compared to biodegradable substrate layer 104. The mask region can provide functional support and can shield an abluminal therapeutic agent from a systemic luminal flow.

In some embodiments, the mask can be relatively impermeable. In some embodiments, permeability can be evaluated using an Endothelial Cell Permeability Assay, whereby differentiated monolayers of endothelial cells are grown on permeable filter supports in order to form tight junctions. Resultant permeability across the cell monolayers can be used to predict human permeability of drug candidates. In some embodiments, a cell permeability assay can include isolating Human Coronary Artery Endothelial Cells (HCAEC), growing the cells to confluence and differentiating the cells for 3 weeks on filters. A test agent, such as a sleeve, is then added to one side of the monolayer, and permeability is assessed by analyzing the concentration of the test agent on the other side of the monolayer using LC/MS. In some embodiments, HCAEC cells have been proven as suitable candidates for the studies of EC (endothelial cell) metabolism and functional vasodilators.

In some embodiments, mask region 107 includes the same polymers as an overlying biodegradable substrate layer 104 but is devoid of therapeutic agents. In some embodiments, mask region includes different polymers than an overlying biodegradable substrate layer 104 and is devoid of therapeutic agents. In some embodiments, the mask region can include a therapeutic agent that is different than the therapeutic agents in an overlying biodegradable substrate layer, such as an endothelial cell growth promoter. A sleeve's composition at different depths can be assessed, for example, using laser ablation mass spectrometry, by visually inspecting the sleeve cross-section using microscopy, using Fourier transform infrared microspectroscopy.

In some embodiments, mask region 107 can have a thickness of 5 nm or more (e.g., 10 nm or more, 20 nm or more, 50 nm or more, 100 nm or more, 500 nm or more, one micron or more, 10 microns or more, 25 microns or more, 50 microns or more, or 70 microns or more) and/or 80 µm or less (e.g., 70 microns or less, 50 microns or less, 25 microns or less, 10 microns or less, one micron or less, 500 nm or less, 100 nm or less, 50 nm or less, 20 nm or less, or 10 nm or less). In some embodiments, mask region 107 can be a uniform layer or patches that may or may not be interconnected. The mask region can fully cover the adluminal surface of an overlying biodegradable substrate layer, such that it can have the same surface area as the adluminal surface of an overlying biodegradable substrate layer.

In some embodiments, tissue-adhesive region 106 can include one or more tissue-adhesive substances. The tissue-adhesive substances can be provided in biodegradable substrate layer 104 (e.g., evenly dispersed in the layer or having a higher concentration at a tissue contacting surface of the layer). In some embodiments, one or more adhesive substances can be provided in an adhesive region that is disposed over the surface of biodegradable substrate layer 104 (which adhesive region may penetrate the biodegradable substrate layer to a certain degree). For example, a pure layer of an adhesive substance or a layer containing an adhesive substance and a suitable adjuvant may be applied to a tissue contacting surface of a biodegradable substrate layer. The tissue-adhesive region allows the sleeve to be in close proximity to the vasculature, reducing the potential for blood leakage through the dissection while providing therapeutic agent delivery to the damaged vasculature.

In some embodiments, the tissue-adhesive region can be configured as a plurality of strips, a plurality of dots, a continuous layer, a matrix mesh, a plurality of longitudinal strips, a plurality of circumferential strips, or any combinations thereof. The tissue-adhesive region can include a repeating pattern of dots and/or strips at predetermined locations. In some embodiments, the tissue adhesive region is disposed over about 5 % or more (e.g., about 10% or more, about 15 % or more, about 20% or more, about 25% or more, about 30% or more, about 50% or more, about 75% or more, or about 90% or more) and about 95 % or less (e.g., about 90% or less, about 75% or less, about 50% or less, about 30% or less, about 25% or less, about 20% or less, about 15% or less, or about 10% or less) of the abluminal surface area of the biodegradable substrate layer that the tissue-adhesive region is disposed on. In some embodiments, the tissue adhesive region can cover greater than 0% up to 100 % of the surface area of an immediately underlying biodegradable substrate layer. In some embodiments, the tissue-adhesive region can seal a biodegradable substrate layer from exposure to a fluid within the body lumen, such that drug elution occurs substantially exclusively (e.g., about 98% or more) to the vascular surface contacting the sleeve. For example, the tissue adhesive region can be located at the ends of the sleeve to minimize leakage of a therapeutic agent from the biodegradable substrate layer. As another example, the tissue adhesive region can be regularly spaced, or at predetermined areas on the biodegradable substrate layer, such as a therapeutic agent is eluted to the vascular area contacting the sleeve. The tissue adhesive region can be porous. In some embodiments, when the tissue adhesive region covers 100% of the underlying biodegradable substrate layer, the adhesive region can protect a therapeutic agent until a treatment site is reached. The surface area of the tissue adhesive region can be dependent on the adhesive properties. In some embodiments, the tissue adhesive region does not delay drug elution from the sleeve.

In some embodiments, the tissue-adhesive region can have a thickness of about 10 nm or more (e.g., about 20 nm or more, about 30 nm or more, about 40 nm or more, about 50 nm or more, about 60 nm or more, about 70 nm or more, about 80 nm or more, or about 90 nm or more) and/or about 100 nm or less (about 90 nm or less, about 80 nm or less, about 70 nm or less, about 60 nm or less, about 50 nm or less, about 40 nm or less, about 30 nm or less, or about 20 nm or less). In some embodiments, the tissue-adhesive region thickness can be influenced by the choice of adhesive. For example, a protein-based adhesive layer can be in the form of a chain of amino acids (a thickness of less than about 10 nm) or can have a thickness that is as large as a sub-micron poly(N-isopropylacrylamide)-based gel particles.

In some embodiments, a tissue adherent strength of a material can be assessed through *in vitro* based peel tests and nano-indentation, which can be used to measure interfacial adhesive properties. For example, nano-indentation data (indicative of Young's modulus and hardness) can be used to correlate thickness with adhesive properties. In some embodiments, the lap-shear strength of a given adhesive can comply with values reported for typical soft-tissue adhesives (about 15 to about 45 kPa).

In some embodiments, tissue adhesive region 106 can improve the therapeutic agent's local proximity to the target treatment area's cellular lining, for example, by decreasing therapeutic agent wash-off to the body lumen. A therapeutic agent 112 can diffuse out of the sleeve to be absorbed by a vessel wall, and the diffusion rate can be controlled by a therapeutic agent concentration within the sleeve and the substrate properties. For example, the ratio of polymer to therapeutic agent can influence the porosity of the sleeve and affect the ability of the therapeutic agent to diffuse out of the matrix. A greater ratio of therapeutic agent can increase the porosity of the sleeve and increase therapeutic agent diffusion. In some embodiments, tissue adhesive region 106 can remain within the body lumen after the clinical procedure is completed. In some embodiments, region 106 can substantially degrade (e.g., degrade by 80 wt% or more, degrade by 90 wt% or more, degrade by 95 wt% or more) before the complete degradation of layer 104, or can substantially degrade after layer 104 has completely degraded.

Tissue-adhesive region can include bioadhesive materials, such as natural polymeric materials, synthetic materials, and synthetic materials formed from biological monomers such as sugars. Tissue adhesives can also be obtained from the secretions of microbes, marine mollusks, and crustaceans. The tissue adhesives can have better adhesion to body tissue, and can have better adhesion to the abluminal surface of a sleeve that the adhesive is attached to rather than to the adluminal surface of an overlying sleeve, or to an overlying release region. In other words, the adhesion at the interface of the sleeve and the carrier balloon is weaker than the adhesion at the interface of the biodegradable substrate layer and the tissue adhesive disposed thereon (or at the interface of the tissue adhesive and the body tissue) so that the biodegradable substrate layer remains with the tissue adhesive region when the carrier balloon is retracted from the body.

In some embodiments, the tissue-adhesive region includes polyethylene glycol, dextran aldehyde, amino acid-based adhesives, adhesive surface proteins, microbial surface components recognizing adhesive matrix molecules ("MSCRAMMS"), fatty ester modified PLA, fatty ester modified PLGA, gel particles, and/or poly(N-isopropylacrylamide) gel particles.

As an example, a polar molecule may be employed as an adhesive substance for the tissue-adhesive region. Examples of such polar molecules include poly(amino acids). For instance, in some embodiments, an amphipathic poly(amino acid) is used as an adhesive substance. The amphipathic poly(amino acid) may have a hydrophobic poly(amino acid) tail (e.g., ranging from 2 to 400 or more amino acids in length) to encourage interaction with the lesion. Examples of hydrophobic amino acids include phenylalanine, leucine, isoleucine and valine, among others. The amphipathic poly(amino acid) may have a hydrophilic poly(amino acid) head (e.g., ranging from 2 to 400 or more amino acids in length) to encourage interaction with the biodegradable polymer (where a hydrophilic polymer such as hyaluronic acid is employed). Examples of hydrophilic amino acids include basic amino acids (e.g., lysine, arginine, histidine, ornithine, etc.), acidic amino acids (e.g., glutamic acid, aspartic acid, etc.), and neutral amino acids (e.g., cysteine, asparagine, glutamine, serine, threonine, tyrosine, glycine). The hydrophilic poly(amino acid) head can be zwitterionic to promote ion-dipole bonding with the biodegradable polymer (where a hydrophilic polymer such as hyaluronic acid is employed). Such a polymer head can contain a mixture of acidic (anionic) and basic (cationic) amino acids and may range, for example, from 2 to 400 or more amino acids in length.

A poly(amino acid) containing a cell-binding peptide such as YIGSR or RGD can be employed as an adhesive substance for the tissue-adhesive region. Such sequences can be repeated if desired. The poly(amino acid) may further comprise a hydrophilic poly(amino acid) chain (e.g., typically ranging from 2 to 400 or more amino acids in length) to promote interaction with the biodegradable polymer (where a hydrophilic polymer such as hyaluronic acid is employed).

In some embodiments, the amino acid 3,4 dihydroxyphenyl alanine (DOPA) or a poly(amino acid) chain that includes multiple DOPA units can be used as an adhesive substance for the tissue-adhesive region. Such chains may further include lysine units, along with the DOPA units. See Statz et al. J. Am. Chem. Soc. 127, 2005, 7972-7973, wherein a 5-mer anchoring peptide (DOPA-Lys- DOPA-LysDOPA) was chosen to mimic the DOPA- and Lys-rich sequence of a known mussel adhesive protein.

In some embodiments, MSCRAMMs (microbial surface components recognizing adhesive matrix molecules) are employed as adhesive substances. Examples of MSCRAMMs include fibronectin binding proteins (e.g., FnBPA, FnBPB, etc.) and fibrinogen binding proteins (e.g., ClfA, ClfB, etc.), among others. See, e.g., Timothy Foster, Chapter 1, "Surface protein adhesins of staphylococci," from Bacterial Adhesion to Host Tissues: Mechanisms and Consequences, Edited by Michael Wilson, 2002, pages 3-11.

In some embodiments, because plaque lesions are known to be hydrophobic, a hydrophobic drug (e.g., paclitaxel, among many others) can be provided over or within the biodegradable polymer containing layer, encouraging adhesion and/or uptake by the lesion upon contact with a lesion.

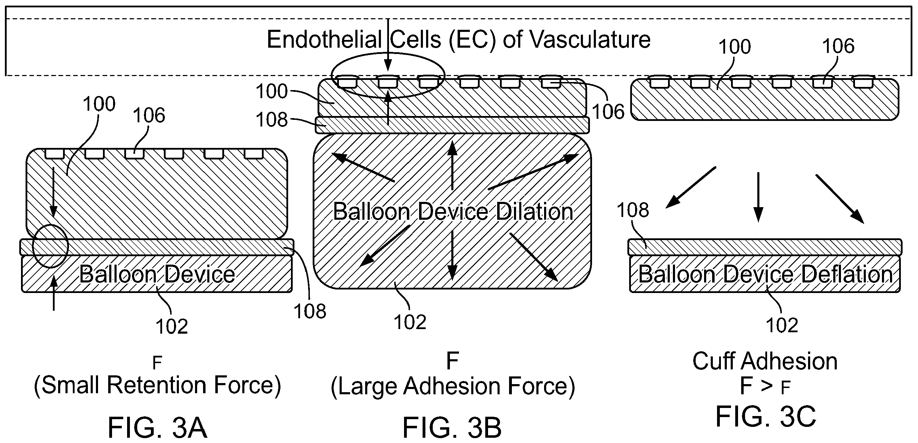

In some embodiments, tissue adhesive region 106 can include hydrogels (e.g., polyethylene glycol:dextran aldehyde) to allow for a strong attractive force to the inner surface of a blood vessel. In some embodiments, referring to FIGS. 3A-3C, the attractive force between adhesive region 106 and a lumen's wall tissue is greater than the retention force between the adluminal surface of the sleeve and the balloon's outer surface, such that the dilation and pressurization of tissue-adhesive region 106 to a lumen's inner surface (e.g., endothelial cell layer, a vascular plaque) sever the retention force between sleeve 100 and balloon 102. Subsequent to balloon deflation and withdrawal, the tissue-adhesive region 106 retains the sleeve in intimate contact with the vasculature. The retention force between sleeve 100 and balloon 102 can result from chemical adhesive forces (e.g., exerted by release region 108) or physical forces (e.g., frictional forces between sleeve 100 and carrier balloon 102).

Referring back to FIG. 1C, balloon 102 can be coated in part or in full with one or more balloon release region(s) 108. Balloon release region 108 can help retain sleeve 100 on balloon 102, which is loaded onto the balloon catheter. Balloon release region can be temporary and biocompatible. For example, balloon release region 108 can include formulations of a contrast agent, such as iopromide (Ultravist ®), which can be used as a contrast medium and as balloon adhesive. In some embodiments, the release region can be configured as a plurality of strips, a plurality of dots, a continuous layer, matrix mesh, a plurality of longitudinal strips, a plurality of circumferential strips, or any combinations thereof. The release region can include a repeating pattern of dots and/or strips, which can be at predetermined locations. In some embodiments, the release region is disposed over 5 % or more (e.g., 10% or more, 15 % or more, 20% or more, 25% or more, 30%, or more, 50% or more, 75% or more, or 90% or more) and/or 95 % or less (90% or less, 75% or less, 50% or less, 30% or less, 25% or less, 20% or less, 15% or less, or 10% or less) of the abluminal surface area of an underlying balloon. In some embodiments, the release region can cover greater than 0% up to 100 % of the surface area of an underlying balloon. The release region can be porous. The surface area of the release region can be dependent on its adhesive and degradation properties.

In some embodiments, specimens with a total surface area of about 0.4 cm² of a gelatin-based biomimetic adhesive can have adhesive strengths of about 12 to about 23 kPa. The adhesive strength can be appropriately adjusted by modulating the extent of the contact surface area.

In some embodiments, the release region can have a thickness of about ten nm or more (e.g., about 20 nm or more, about 30 nm or more, about 40 nm or more, about 50 nm or more, about 60 nm or more, about 70 nm or more, about 80 nm or more, or about 90 nm or more) and/or about 100 nm or less (about 90 nm or less, about 80 nm or less, about 70 nm or less, about 60 nm or less, about 50 nm or less, about 40 nm or less, about 30 nm or less, or about 20 nm or less).

In some embodiments, the release region can include contrast agents (e.g., iopromide), proteins (e.g., gelatin-based glues, protein-based adhesives), synthetic glues (e.g., cyanoacrylates), or any combination thereof. For example, the release region can include gelatin-based glues (e.g., resorbable biological glues such as GRFG - gelatin, resorcinol, formaldehyde, glutaraldehyde), gelatin hydrogel glues, cyanoacrylates (e.g. Histoacryl blue), adhesive based on protein engineering (e.g., high grade biocompatibility and biodegradability internal adhesives). In some embodiments, for better retention of the release region on a balloon surface during delivery, the release region can include crosslinked gel particles, or the gel particles can be mixed with a higher molecular weight polymer.

In some embodiments, the balloon surface can have "windows" that can allow for release of a physico-mechanical signal across the "window" to facilitate sleeve detachment. In some embodiments, a "window" can include a hole, an aperture, a pore, a thinner area of the same polymer, and/or a membrane of an alternate material. For example, the windows can enable the transfer of a detachment agent (e.g., a change in temperature, a change in pH) across the window when the deployment balloon has been flushed with an appropriate catalyst. The catalyst can include an external agent, which can be physical or chemical in nature. As an example, a cryo-technique such as that used in the cryo-catheter devices can deliver extreme cold (e.g., a catalyst) from the tip of an ablation catheter or through a balloon. As another example, heat (e.g., a catalyst) can be applied in the same manner as extreme cold. In some embodiments, adhesion can be regulated through modulation of pH. For example, the availability of local calcium ions (a catalyst) can be adjusted and used to vary alkaline balance.

The catalyst can initiate localized site degradation of a window, when the window is, for example, a thinner area of the same polymer or of a membrane of an alternate material. In some embodiments, the catalyst can initiate the degradation of a balloon adhesive to allow a sleeve to be detached and deployed at a treatment site. The catalyst can be released through, for example, a hole, an aperture, a pore, a thinner area of the same polymer, or a membrane of an alternate material.

In some embodiments, one or more release substances can be provided in a release region that is disposed between the surfaces of the carrier balloon (or an underlying sleeve) and an overlying sleeve. The release region may penetrate the immediately overlying sleeve (e.g., a mask layer 107 of an overlying sleeve) to a certain degree. As an example, a release substance can include zwitterionic phosphorylcholine and its derivatives. Without wishing to be bound by theory, it is believed that phosphorylcholine can form ionic-dipole bonds with various polar substances, including biodegradable polymers such as hyaluronic acid and polar balloon materials such as PEBAX. Therefore, phosphorylcholine can bind the biodegradable polymer portion of the sleeve to the balloon material. In some embodiments, a wetting agent (e.g., saline or water) can be employed to disrupt the ionic-dipole interactions holding the sleeve on the balloon.

In some embodiments, the wetting agent is supplied by the delivery vehicle (e.g., a delivery balloon). For example, an inflatable micro-porous or weeping balloon may be used to dilate the vessel site and deliver a wetting agent which can interact with zwitterionic phosphorylcholine. As another example, saline loaded microspheres can be provided between an overlying sleeve and the balloon, which can burst and release their contents upon balloon inflation.

Other zwitterionic materials may be employed as release substances including zwitterionic peptides. For example, peptides with both basic amino acids (e.g., lysine, arginine, ornithine, etc.) and acidic amino acids (e.g., glutamic acid, aspartic acid, etc.) will have zwitterionic character for providing ionic ionic-dipole bonds with various polar substances (e.g., a hydrophilic biodegradable polymer or a hydrophilic balloon material). Chains of non-polar amino acid chains (e.g., phenylalanine, leucine, isoleucine, valine, etc.) may be attached to zwitterionic chains for providing hydrophobic interactions with various nonpolar substances (e.g., a hydrophobic balloon material).

Shear sensitive adhesives constitute another class of release substance that may be used between a balloon delivery vehicle and a sleeve. The basic principle of these adhesives is that the shearing force that is created between the inflating balloon and the adhesive will break the bond and facilitate release. An example of such an adhesive is a blend of polyvinylpyrrolidone (PVP) and polyethylene glycol (PEG), which would provide a biocompatible layer which adheres the balloon to the biodegradable substrate layer until the device is in place at the delivery site. Balloon dilation may be used to disrupt the adhesive bonds and the sleeve may thus be released from the balloon. The weight ratio of PVP to PEG in such blends may vary widely, for example, ranging from 1:99 to 10:90 to 25:75 to 50:50 to 75:25 to 90:10 to 95:5 to 99:1.

In some embodiments, the release region can remain attached to the adluminal surface of an implanted overlying sleeve and can degrade with the overlying sleeve in a body lumen. Degradation of the release region can occur in a controlled manner. For example, degradation of the release region can be completed in three months or less (e.g., two months or less, one month or less, two weeks or less, one week or less, three days or less, two days or less, one day or less, twelve hours or less, six hours or less, one hour or less, 30 minutes or less, 15 minutes or less, 5 minutes or less, one minute or less) and/or 30 seconds or more (e.g., one minute or more, 5 minutes or more, 15 minutes or more, 30 minutes or more, one hour or more, six hours or more, twelve hours or more, one day or more, two days or more, three days or more, on week or more, two weeks or more, one month or more, or two months or more). This degradation profile can be designed to be of short duration if a release region has already fulfilled its primary function. In some embodiments, the release region degradation can be matched to the duration of the sleeve degradation.

In some embodiments, the sleeve can also offer an opportunity for corrective measures. For example, if unforeseen malposition or vascular blockage is caused by a sleeve during use, the introduction of an appropriate bolus of suitable fluid to the locality of the problematic sleeve can be used to accelerate its degradation and thus return a blood vessel to its unblocked state. Treatment can be therefore administered without full interventional procedures (e.g., surgical intervention). Without wishing to be bound by theory, it is believed that malposition or vascular blockage can have an increased risk of occurrence in procedures involving distal vasculature with small lumen diameter. In some embodiment, depending on the composition of the sleeve, accelerated degradation of a sleeve can include flushing a body lumen with a saline solution, changing the pH of the local environment of a sleeve, administering cryo-treatment to the local environment of a sleeve, and/or administering ultrasound to the local environment of a sleeve. Accelerated degradation can occur over the period of one month or less (e.g., three weeks or less, two weeks or less, one week or less, three days or less, one day or less, 12 hours or less, six hours or less, one hour or less, 30 minutes or less, 15 minutes or less, five minutes or less, or one minute or less). As an example, a sleeve can include a 200 µm thick film of about 85/15 lactide:glycolide PLGA co-polymer, and *in vitro* mass loss tests can be conducted at 37 °C in bio-relevant media. Greater than 85% mass loss of the film can occur in less than 180 days. These findings are supported, for example, by preclinical studies. As another example, a sleeve can include a 200 µm thick film of 50/50 lactide:glycolide PLGA co-polymer, and *in vitro* mass loss studies can be conducted at 37 °C in bio-relevant media. Greater than 90% mass loss of the film can occur in less than 145 days. These findings are supported, for example, by preclinical studies.

A wide variety of therapeutic agents may be used in the sleeves. A therapeutic agent may be used singly or in combination with other therapeutic agents. The terms "therapeutic agent", "pharmaceutically active agent", "pharmaceutically active material", "pharmaceutically active ingredient", "drug", "beneficial agent", "bioactive agent" and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. By small organic molecule is meant an organic molecule having 50 or fewer carbon atoms, and fewer than 100 non-hydrogen atoms in total. Generally, exemplary therapeutic agents include, e.g., sirolimus, everolimus, biolimus (e.g., biolimus A9), zotarolimus, tacrolimus and paclitaxel. The therapeutic agent can be amorphous.

Exemplary therapeutic agents can also include coagulant agents, antianginals, and analgesics. Examples of antianginals can include nitrates, beta blockers, and calcium channel blockers. Example of analgesics can include Paracetamol and NSAIDs, COX-2 inhibitors, and Flupirtine. Examples of coagulant agents/promoters include promoters of the expression of human coagulation factors (II,IV,VI, VIII and C) or procoagulant drugs, such as desmopressin.

In some embodiments, exemplary non-genetic therapeutic agents include anti-thrombogenic agents such as heparin and derivatives, prostaglandin, urokinase, and PPack (dextrophenylalanine proline arginine chloromethylketone); anti-proliferative agents such as enoxaparin and angiopeptin, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, azathioprine, doxorubicin, daunorubicin, cyclosporine, mitomycin, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as thylenediaminetetraacetic acid, O,O'-bis (2-aminoethyl) ethyleneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, antithrombin compounds, platelet receptor antagonists, anti-thrombin antibodies, anti-platelet receptor antibodies, enoxaparin, hirudin, warfarin sodium, Dicumarol, prostaglandin inhibitors, platelet aggregation inhibitors such as cilostazol and tick antiplatelet factors; vascular cell growth promoters such as growth factors, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; protein bound particle drugs such as ABRAXANE™; structural protein (*e.g*., collagen) cross-link breakers such as alagebrium (ALT-711); and/or any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMPs"), such as, for example, BMP-2, BMP-3, BMP-4, BMP-5, BMP-6 (VGR-1), BMP-7 (OP-1), BMP-8, BMP-9, BMP-10, BMPs-11, BMP-12, BMP-13, BMP-14, and BMP-15. Preferred BMPs are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNAs encoding them. Non-limiting examples of genes include survival genes that protect against cell death, such as antiapoptotic Bcl-2 family factors and Akt kinase; serca 2 gene; and combinations thereof.

Any of the therapeutic agents may be combined to the extent such combination is biologically compatible.

Examples of medical devices benefiting from use in conjunction with the present disclosure vary widely and can include implantable or insertable medical devices, for example, stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (e.g., urological catheters or vascular catheters such as balloon catheters and various central venous catheters), guide wires, balloons, filters (e.g., vena cava filters and mesh filters for distil protection devices), embolization devices including cerebral aneurysm filler coils (including Guglielmi detachable coils and metal coils), septal defect closure devices, myocardial plugs, patches, electrical stimulation leads, including leads for pacemakers, leads for implantable cardioverter-defibrillators, leads for spinal cord stimulation systems, leads for deep brain stimulation systems, leads for peripheral nerve stimulation systems, leads for cochlear implants and leads for retinal implants, ventricular assist devices including left ventricular assist hearts and pumps, total artificial hearts, shunts, valves including heart valves and vascular valves, anastomosis clips and rings, tissue bulking devices, and tissue engineering scaffolds for cartilage, bone, skin and other in vivo tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, or other devices that are implanted or inserted into the body and from which therapeutic agent is released.

In some embodiments, suitable medical devices on which a sleeve may be carried include, but are not limited to, those that have a tubular or cylindrical like portion. A tubular portion of a medical device need not be completely cylindrical. The cross-section of the tubular portion can be any shape, such as rectangle, a triangle, etc., not just a circle. Such devices include, but are not limited to, stents, balloons of a balloon catheters, grafts, and valves (e.g., a percutaneous valve). A bifurcated stent is also included among the medical devices which can be fabricated by the methods described herein. The device can be made of any material, e.g., metallic, polymeric, and/or ceramic material.

In some embodiments, examples of balloon materials include relatively non-complaint materials such as polyamides, for instance, polyamide homopolymers and copolymers and composite materials in which a matrix polymer material, such as polyamide, is combined with a fiber network (e.g., Kevlar®: an aramid fiber made by Dupont or Dyneema®, a super-strong polyethylene fiber made by DSM Geleen, the Netherlands). Specific examples of poly amides include nylons, such as nylon 6, nylon 4/6, nylon 6/6, nylon 6/10, nylon 6/12, nylon 11 and nylon 12 and poly(ether-coamide) copolymers, for instance, polyether-polyamide block copolymer such as poly(tetramethylene oxide-b-polyamide-12) block copolymer, available from Elf Atochem as PEBAX. Examples of balloon materials also include relatively complaint materials such as silicone, polyurethane or compliant grades of PEBAX having a larger percentage of poly ether, for example PEBAX 63D. In some embodiments, examples of balloon materials can include semi-compliant polymer materials.

### Method of Manufacture

Where the delivery device is a balloon, the sleeve may be applied to a folded balloon to minimize interactions between the device and the balloon that would have to be disrupted for device delivery, thereby improving release. In some embodiments, sleeves can be made and then applied to a delivery device. For example, a drug delivery sleeve comprising an inner mask region, a drug-releasing biodegradable fibrous layer, and an outer adhesive region may be formed and applied to a balloon, which may be folded in certain embodiments.

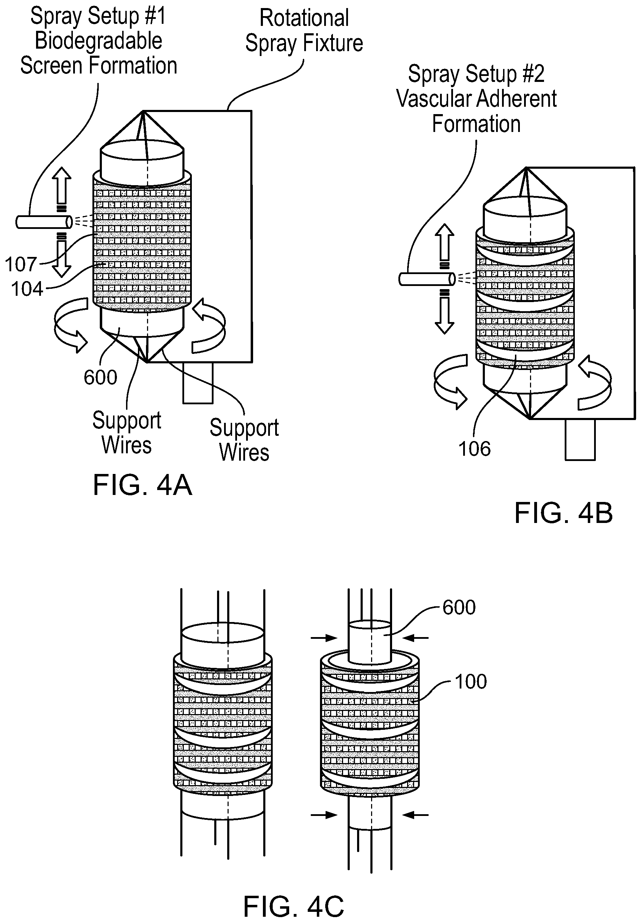

In some embodiments, the balloon is manufactured by extrusion technology. Referring to FIGS. 4A, mask region 107 and biodegradable substrate layer 104 can be formed by sequentially spraying or otherwise applying (e.g., dipping, brushing, painting) a polymer solution, followed by a polymer and therapeutic agent solution, to a cylindrical non-stick template 600 (e.g., a poly(tetrafluoroethylene) "PTFE" template). Once the mask region and biodegradable substrate layer are formed, the tissue-adhesive region 106 can be applied to the substrate layer, in a selective manner at predefined patterns, as illustrated in FIG. 4B. Tissue adhesive region 106 can be applied, for example, by dipping, spraying, dropping-on-demand, and/or roll-coating. Upon completion of sleeve formation, the coating apparatus can be dismantled to leave the sleeve 100, which can be removed from the PTFE template 600, as illustrated in FIG. 4C. The PTFE template can be reused.

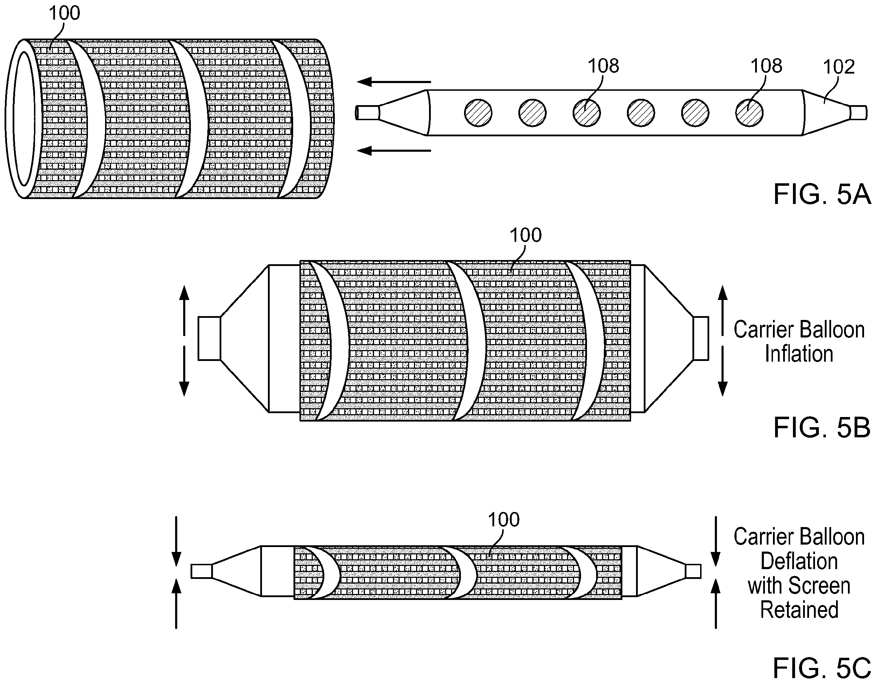

Referring to FIG. 5A, the sleeve 100 can then be introduced over a carrier balloon 102, which can have pre-applied balloon release region 108. Referring to FIG. 5B, the sleeve can be fitted over the carrier balloon, and the carrier balloon can be subsequently inflated to cause a mechanical interaction between the balloon and the sleeve, thereby allowing the release layer to be in contact with the sleeve. Referring to FIG. 5C, deflation can cause the sleeve to follow the balloon to which it is now adhered to and to reform the underlying folded shape of the balloon.

In some embodiments, the sleeve includes an elastomeric material so that it can be placed and folded with a balloon carrier, such that when expanded in a treatment site, plastic deformation of the sleeve can be permanently induced. During the balloon folding process, the sleeve is sized to conform to the balloon diameter, ensuring that post balloon folding, a reduced profile is achieved. In some embodiments, no additional balloon-adhesion region is required.

In some embodiments, rather than an elastomeric material that would wrap around and be folded along with the balloon, a tube of elastomeric material having tissue-adhesive region can be sized such that it can be placed over a folded balloon. In this case, the sleeve and the balloon can have a smaller assembly profile, as the sleeve is not folded together with the balloon. The sleeve can expand with the unfolding balloon during inflation, which can induce plastic deformation of the sleeve, and the sleeve can remain and adhere at the inflated diameter within a treatment site after removal of the balloon catheter.

In some embodiments, the balloon cones can be puffed (e.g., during packaging and/or device delivery) to ensure securement of the sleeve.

Optionally, a stent may be provided (a) before application of the sleeve (in the event an abluminal sleeve is desired for the stent) or (b) after application of the sleeve (in the event an adluminal sleeve is desired for the stent). As another example, a drug delivery sleeve comprising an inner release region, a first drug-releasing biodegradable substrate layer, a mask region, a second drug-releasing biodegradable substrate layer, and an outer adhesive region may be formed and applied to a balloon, which may be folded in certain embodiments. Different drugs may be supplied in the fibrous layers, for example, an endothelial cell growth promoter may be provided in the inner adlumenal biodegradable substrate layer and a coagulant may be provided in the outer ablumenal biodegradable substrate layer.

In other embodiments, sleeves may be formed on the surface of the delivery device. As a specific example (among many other possibilities), a release region may first be applied to a surface of an inflatable balloon. A mask region containing a polymer can be applied on the release region, a biodegradable polymer containing layer including a therapeutic agent is then formed over the mask region. In a subsequent step, an adhesive region is provided over the biodegradable substrate layer. As a more specific example, a release region may first be applied to a surface of an inflatable balloon formed from a material such as nylon, polyurethane or PEBAX, among others. The release region may comprise, among other possibilities, (a) a shear sensitive adhesive or (b) a zwitterionic release substance such as phosphorylcholine in combination with saline microcapsules (unless a micro-porous or weeping balloon is employed, in which case the saline microcapsules will be excluded). A mask region comprising hyaluronic acid is formed over the release layer. A biodegradable substrate layer, for example, comprising hyaluronic acid and paclitaxel as a therapeutic agent is then formed over the mask region, for instance, via a spraying process. The hyaluronic acid layers may then be crosslinked by applying genipin to the layers. In a subsequent step, DOPA is applied to the outer fiber layer surface as an adhesive substance, among other possibilities.

Optionally, a stent may be provided (a) before application of mask and biodegradable substrate layer (in the event an abluminal sleeve is desired), (b) after application of the mask and biodegradable substrate layer (in the event a adluminal sleeve is desired) or (c) after application of a mask and biodegradable substrate layer, followed by formation of another biodegradable substrate layer layer (in the event that a biodegradable substrate layer-encapsulated stent structure with adluminal and abluminal biodegradable substrate layers is desired).

As noted above, examples of mask layers can include non-porous layers, and examples of biodegradable substrate layers include non-porous layers (e.g., hydrogel layers) and porous layers (e.g., fibrous layers). Non-porous layers may be provided using techniques such as by dipping, spray coating, coating with an applicator (e.g., by roller, brush, etc), and so forth.

Fibrous layers may be formed using, for example, fiber spinning techniques. For example, electrospinning is a fiber spinning technique by which a suspended drop of polymer (e.g., a polymer in a suitable solvent) is charged with tens of thousands of volts. At a characteristic voltage, the droplet forms a Taylor cone, and a fine jet of polymer releases from the surface in response to the tensile forces generated by interaction of an applied electric field with the electrical charge carried by the jet. This produces a filament of material. This jet can be directed to a grounded surface such as a balloon delivery system and collected as a continuous web of fibers that can be adjusted to give fibers ranging in size, for example, from 50 nm to 100 nm to 250 nm to 500 nm to 1 micron to 2.5 microns to 5 microns to 10 microns to 20 microns. To ensure good coverage, the balloon delivery system may be rotated and reciprocated relative to the jet. Multiple dispensers with differing concentrations of starting materials may be utilized to produce higher concentrations of selected materials in specific areas of the nanofibrous network. Further information on electrospinning may be found, for example, in US 2005/0187605 to Greenhalgh et al. See also Y. Ji et al., "Electrospun three-dimensional hyaluronic acid nanofibrous scaffolds," Biomaterials 27 (2006) 3782-3792.

Porous layers including electrospun fibrous layers increase available surface area and therefore may increase release of any therapeutic agents and increase biodegradation rate relative to nonporous layers. Moreover, such layers may serve to create a scaffold for cell seeding, growth and/or proliferation. For example, in the case of vascular devices, such layers may serve as a scaffold for endothelial cell seeding, growth and/or proliferation *in vivo.*

In some embodiments, it may be desirable to roughen a surface of interest before performing the depositions as described herein. For example, a surface may be roughened to provide a series of nooks or invaginations on/within the surface. Any surface may be roughened, e.g., a metallic, polymeric or ceramic surface. Surfaces can be roughened using any technique known in the art. Particularly useful methods for roughening surfaces, such as the surfaces of a stent, are described, e.g., in U.S. Serial No. 12/205,004, which is hereby incorporated by reference. The surface of a balloon may also be roughened.

Further, as will be appreciated by skilled practitioners, mask region and biodegradable substrate layer can be deposited on an entire surface of a template or onto only part of a surface of the template. This can be accomplished using a separate template-shielding mask to shield the portions on which coatings are not to be deposited. In some embodiments, the template is a stent. It may be desirable to deposit only on the abluminal surface of the stent. This construction may be accomplished by, e.g. coating the stent before forming the fenestrations. In other embodiments, it may be desirable to deposit only on abluminal and "cutface" surfaces of the stent. This construction may be accomplished by, e.g., depositing on a stent containing a mandrel, which shields the luminal surfaces.

In various embodiments, one or more therapeutic agents may also be included, for example, along with one or more biodegradable polymers in a solution that is used to form a biodegradable substrate layer. As an alternative, a biodegradable polymer and one or more therapeutic agents may be simultaneously deposited (e.g., from separate containers) to form a biodegradable substrate layer. As another alternative, one or more therapeutic agents may be applied (e.g., in solution) to the biodegradable substrate layer after it is formed.

### Examples

### Example 1

A sleeve is composed of a mask layer and a biodegradable substrate layer. The mask layer assembly is formed of an extruded poly(glycolide-lactide) copolymer. A porous biodegradable substrate layer is formed of a low dose of desmopressin/poly-DL-lactide in a 10:90 composition. The design has a nominal dosage of 2 µg/mm² desmopressin. The porous substrate layer can be formed through electro-spinning.

The sleeve has a tissue adherent layer formed of a poly (N-isopropylacrylamide) gel matrix and a balloon adherent layer composed of a gelatin hydrogel glue. The tissue adhesive region is configured as a plurality of dots and is applied by drop-on-demand technology.

### Example 2

A sleeve is formed of a mask layer and a biodegradable substrate layer. The mask layer assembly is formed of an extruded poly(glycolide-lactide) copolymer. The biodegradable substrate layer is formed of a fibrinogen/poly-DL-lactide network derived from immersion of a poly-DL-lactide polymeric mesh in a 1 mg/ml fibrinogen solution. The design has a nominal dosage of 1 ng/mm² of protein immobilized on the surface devised with a strong covalent binding between the protein and the substrate.

The sleeve has a tissue adherent layer formed of a polyethylene glycol matrix and a balloon adherent layer formed of a gelatin hydrogel glue. The tissue adhesive region is configured as a plurality of strips and is applied by drop-on-demand technology. The balloon/sleeve adhesive regions are configured as a plurality of strips and are applied by drop-on-demand technology.

All references, such as patent applications, publications, and patents, referred to herein are incorporated by reference in their entirety.

A number of embodiments of the disclosure have been described. Nevertheless, it will be understood that various modifications may be made without departing from the scope of the disclosure. Accordingly, other embodiments are within the scope of the following claims.

## Claims

1. A medical device, wherein the medical device is a vascular sleeve and can be disposed on an expandable balloon, comprising:
a tubular layer having an adluminal surface and an abluminal surface, the layer comprising a first polymer and a biologically active agent;
a tissue-adhesive region disposed on the outer abluminal surface; and
a mask region comprising a second polymer disposed on the inner adluminal surface.

2. The medical device of claim 1, wherein the mask region is non-porous.

3. The medical device of claim 1, wherein the device is biodegradable, in particular wherein the device is biodegradable within a period of about one month to about twelve months.

4. The medical device of claim 1, wherein the first and second polymers are each independently selected from the group consisting of polyurethanes, polyethylene, polylactic acid, polyglycolic acid, polylactic-co-glycolic acid, poly-DL-lactide, and any combination thereof.

5. The medical device of claim 1, wherein the tissue-adhesive region is configured on the abluminal surface as a plurality of strips, a plurality of dots, a continuous layer, a matrix mesh, a plurality of longitudinal strips, a plurality of circumferential strips, or any combination thereof.

6. The medical device of claim 5, wherein the tissue-adhesive region comprises a repeating pattern of dots, strips, or both.

7. The medical device of claim 5, wherein the tissue-adhesive region is disposed at a first end and second end of the tubular layer.

8. The medical device of claim 1, further comprising a release region disposed between the medical device and the surface of the expandable balloon.

9. The medical device of claim 8, wherein the release region is adherent to the expandable balloon.

10. The medical device of claim 1, wherein the tissue adhesive region is disposed over about 5 percent or more of the abluminal surface of the tubular layer.

11. The medical device of claim 8, wherein the release region comprises about 5 percent or more of the adluminal surface area of the mask layer.

12. The medical device of claim 1, wherein the tissue-adhesive region comprises polyethylene glycol, dextran aldehyde, amino acid-based adhesives, adhesive surface proteins, MSCRAMMS, fatty acid modified PLA, fatty acid modified PLGA, gel particles, poly(N-isopropylacrylamide) gel particles, or any combination thereof.

13. The medical device of claim 8, wherein the release layer comprises contrast agents, proteins, synthetic glues, or any combination thereof.

14. The medical device of claim 1, wherein the biologically active agent is selected from the group consisting of antianginals, analgesics, nitrates, beta blockers, calcium channel blockers, paracetamol, NSAIDs, COX-2 inhibitors, flupirtine, coagulant promotors, procoagulants, Desmopressin, paclitaxel, everolimus, sirolimus, zotarolimus, biolimus A9, and any combination thereof.

15. A method of making a medical device, wherein the medical device is a vascular sleeve and can be disposed on an expandable balloon, comprising:
(a) applying a layer comprising a second polymer to a non-stick substrate to form a substantially non-porous mask region;
(b) applying a tubular layer comprising a first polymer and biologically active agent to the mask region;
(c) applying a tissue-adhesive region to the tubular layer to form a sleeve;
(d) removing the sleeve from non-stick substrate; and
(e) disposing the sleeve over an expandable balloon coated with a first release agent.

## Patentansprüche

1. Medizinische Vorrichtung, wobei die medizinische Vorrichtung eine Gefäßmanschette ist und auf einem expandierbaren Ballon angeordnet werden kann, umfassend:
eine rohrförmige Schicht mit einer adluminalen Oberfläche und einer abluminalen Oberfläche, wobei die Schicht ein erstes Polymer und ein biologisch aktives Mittel umfasst;
einen gewebehaftenden Bereich, der an der äußeren abluminalen Oberfläche angeordnet ist; und
einen Maskenbereich, umfassend ein zweites Polymer, das auf der inneren adluminalen Oberfläche angeordnet ist.

2. Medizinische Vorrichtung gemäß Anspruch 1, wobei der Maskenbereich nichtporös ist.

3. Medizinische Vorrichtung gemäß Anspruch 1, wobei die Vorrichtung biologisch abbaubar ist, insbesondere wobei die Vorrichtung innerhalb eines Zeitraums von etwa einem Monat bis etwa zwölf Monaten biologisch abbaubar ist.

4. Medizinische Vorrichtung gemäß Anspruch 1, wobei das erste und das zweite Polymer jeweils unabhängig ausgewählt sind aus der Gruppe bestehend aus Polyurethanen, Polyethylen, Polymilchsäure, Polyglycolsäure, Polymilchsäure-co-glycolsäure, Poly-DL-lactid und beliebigen Kombinationen davon.

5. Medizinische Vorrichtung gemäß Anspruch 1, wobei der gewebehaftende Bereich auf der abluminalen Oberfläche als Vielzahl von Streifen, Vielzahl von Punkten, kontinuierliche Schicht, Matrixnetz, Vielzahl von Längsstreifen, Vielzahl von Umfangsstreifen oder beliebige Kombination davon gestaltet ist.

6. Medizinische Vorrichtung gemäß Anspruch 5, wobei der gewebehaftende Bereich ein Wiederholungsmuster von Punkten, Streifen oder beidem umfasst.

7. Medizinische Vorrichtung gemäß Anspruch 5, wobei der gewebehaftende Bereich an einem ersten Ende und einem zweiten Ende der rohrförmigen Schicht angeordnet ist.

8. Medizinische Vorrichtung gemäß Anspruch 1, ferner umfassend einen Freigabebereich, der zwischen der medizinischen Vorrichtung und der Oberfläche des expandierbaren Ballons angeordnet ist.

9. Medizinische Vorrichtung gemäß Anspruch 8, wobei der Freigabebereich an dem expandierbaren Ballon haftet.

10. Medizinische Vorrichtung gemäß Anspruch 1, wobei der gewebehaftende Bereich über etwa 5 Prozent oder mehr der abluminalen Oberfläche der rohrförmigen Schicht angeordnet ist.

11. Medizinische Vorrichtung gemäß Anspruch 8, wobei der Freigabebereich etwa 5 Prozent oder mehr der adluminalen Oberfläche der Maskenschicht umfasst.

12. Medizinische Vorrichtung gemäß Anspruch 1, wobei der gewebehaftende Bereich Polyethylenglycol, Dextranaldehyd, Klebstoffe auf Aminosäurebasis, adhäsive Oberflächenproteine, MSCRAMMS, fettsäuremodifizierte PLA, fettsäuremodifizierte PLGA, Gelpartikel, Poly(N-isopropylamid)-Gelpartikel oder eine beliebige Kombination davon umfasst.

13. Medizinische Vorrichtung gemäß Anspruch 8, wobei die Freigabeschicht Kontrastmittel, Proteine, synthetische Klebstoffe oder eine beliebige Kombination davon umfasst.

14. Medizinische Vorrichtung gemäß Anspruch 1, wobei das biologisch aktive Mittel ausgewählt ist aus der Gruppe bestehend aus Antianginosa, Analgetika, Nitraten, Betablockern, Calciumkanalblockern, Paracetamol, NSAIDs, COX-2-Hemmern, Flupirtin, Koagulationsförderern, Prokoagulantien, Desmopressin, Paclitaxel, Everolimus, Sirolimus, Zotarolimus, Biolimus A9 und einer beliebigen Kombination davon.

15. Verfahren zum Herstellen einer medizinischen Vorrichtung, wobei die medizinische Vorrichtung eine Gefäßmanschette ist und auf einem expandierbaren Ballon angeordnet werden kann, umfassend:
(a) Aufbringen einer Schicht, die ein zweites Polymer umfasst, auf ein nichthaftendes Substrat, um einen im Wesentlichen nichtporösen Maskenbereich zu bilden;
(b) Aufbringen einer rohrförmigen Schicht, die ein erstes Polymer und ein biologisch aktives Mittel umfasst, auf den Maskenbereich;
(c) Aufbringen eines gewebehaftenden Bereichs auf die rohrförmige Schicht, um eine Manschette zu bilden;
(d) Entfernen der Manschette von dem nichthaftenden Substrat; und
(e) Anordnen der Manschette über einem expandierbaren Ballon, der mit einem ersten Freigabemittel beschichtet ist.

## Revendications

1. Dispositif médical, où le dispositif médical est un manchon vasculaire et peut être disposé sur un ballonnet expansible, comprenant : une couche tubulaire présentant une surface adluminale et une surface abluminale, la couche comprenant un premier polymère et un agent biologiquement actif ; une région adhérant aux tissus disposée sur la surface abluminale extérieure ; et une région de masque comprenant un deuxième polymère disposé sur la surface adluminale intérieure.

2. Dispositif médical selon la revendication 1, où la région de masque est non poreuse.

3. Dispositif médical selon la revendication 1, où le dispositif est biodégradable, en particulier où le dispositif est biodégradable en une durée d'environ un mois à environ douze mois.

4. Dispositif médical selon la revendication 1, où chacun des premier et deuxième polymères est indépendamment choisi dans le groupe constitué par les suivants :
polyuréthanes, polyéthylène, acide polylactique, acide polyglycolique, acide polylactique-co-glycolique, poly-DL-lactide, et n'importe laquelle de leurs combinaisons.

5. Dispositif médical selon la revendication 1, où la région adhérant aux tissus est configurée sur la surface abluminale sous forme d'une multitude de bandes, d'une multitude de points, d'une couche continue, d'une maille matricielle, d'une multitude de bandes longitudinales, d'une multitude de bandes circonférentielles, ou n'importe laquelle de leurs combinaisons.

6. Dispositif médical selon la revendication 5, où la région adhérant aux tissus comprend un motif de répétition en points, bandes, ou les deux.

7. Dispositif médical selon la revendication 5, où la région adhérant aux tissus est disposée au niveau d'une première extrémité et d'une deuxième extrémité de la couche tubulaire.

8. Dispositif médical selon la revendication 1, comprenant en outre une région de démoulage disposée entre le dispositif médical et la surface du ballonnet expansible.

9. Dispositif médical selon la revendication 8, où la région de démoulage adhère au ballonnet expansible.

10. Dispositif médical selon la revendication 1, où la région adhérant aux tissus est disposée par-dessus environ 5 pour cent ou plus de la surface abluminale de la couche tubulaire.

11. Dispositif médical selon la revendication 8, où la région de démoulage constitue environ 5 pour cent ou plus de la zone de surface adluminale de la couche de masque.

12. Dispositif médical selon la revendication 1, où la région adhérant aux tissus comprend les suivants : polyéthylène glycol, dextrane aldéhyde, adhésifs à base d'acides aminés, protéines de surface adhésive, MSCRAMMS, PLA modifié par acides gras, PLGA modifié par acides gras, particules de gel, particules de gel de poly(N-isopropylacrylamide), ou n'importe laquelle de leurs combinaisons.

13. Dispositif médical selon la revendication 8, où la couche de démoulage comprend des agents de contraste, des protéines, des colles synthétiques, ou n'importe laquelle de leurs combinaisons.

14. Dispositif médical selon la revendication 1, où l'agent biologiquement actif est choisi dans le groupe constitué par les suivants : antiangineux, analgésiques, nitrates, bêta-bloquants, agents bloquants des canaux calciques, paracétamol, AINS, inhibiteurs de COX-2, flupirtine, promoteurs de coagulation, procoagulants, Desmopressine, paclitaxel, évérolimus, sirolimus, zotarolimus, biolimus A9, et n'importe laquelle de leurs combinaisons.

15. Procédé de fabrication d'un dispositif médical, où le dispositif médical est un manchon vasculaire et peut être disposé sur un ballonnet expansible, comprenant :
(a) l'application d'une couche comprenant un deuxième polymère à un substrat non collant pour former une région de masque substantiellement non poreuse ;
(b) l'application d'une couche tubulaire comprenant un premier polymère et un agent biologiquement actif à la région de masque ;
(c) l'application d'une région adhérant aux tissus à la couche tubulaire pour former un manchon ;
(d) le retrait du manchon d'avec le substrat non collant ; et
(e) la disposition du manchon par-dessus un ballon expansible revêtu d'un premier agent de démoulage.
